# EUROPEAN PATENT APPLICATION

(11) **EP 4 831 158 A1**
(43) Date of publication of application: **28.10.2026**
(21) Application number: 26172993.3
(22) Date of filing: 17.04.2026
(51) Int. Cl.: C07C 303/26, C07C 17/263, C07C 29/36

(54) **PROCESS FOR PREPARING 1-ARENESULFONYLOXY-5-HALOALKANE COMPOUND AND PROCESS FOR PREPARING 6,10,14-TRIMETHYL-2-PENTADECANOL THEREFROM**

(30) Priority: 24.04.2025 JP 2025072458
(71) Applicant: SHIN-ETSU CHEMICAL CO., LTD., Tokyo 100-0005 (JP)
(72) Inventor: MIYAKE, Yuki, Niigata, 942-8601 (JP); WATANABE, Takeru, Niigata, 942-8601 (JP)
(74) Representative: De Vries & Metman

(57) **Abstract**

The present invention relates to a process for preparing a 1-arenesulfonyloxy-5-haloalkane compound (3), wherein R¹ and R² represent, independently of each other, a hydrogen atom or a methyl group, and X¹ represents a halogen atom, the process comprising a step of subjecting a tetrahydropyran compound (1), wherein R¹ represents a hydrogen atom or a methyl group, to a ring-opening reaction with an arenesulfonyl halide compound (2), wherein R² represents a hydrogen atom or a methyl group, and X¹ represents a halogen atom, in the presence of zinc chloride to form the aforesaid 1-arenesulfonyloxy-5-haloalkane compound (3).

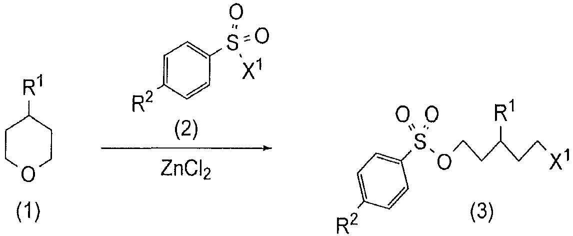

## Description

### TECHNICAL FIELD

An embodiment of the present invention relates to a process for preparing a 1-arenesulfonyloxy-5-haloalkane compound, and a process for preparing 6,10,14-trimethyl-2-pentadecanol therefrom.

### BACKGROUND ART

1-Arenesulfonyloxy-5-haloalkane compounds can construct long-chain hydrocarbon skeletons via coupling reactions, and are therefore extremely useful as synthesis precursors of insect pheromones and insect cuticular hydrocarbon compounds having methyl branches. Known insect pheromones having methyl branches include, for example, the sex pheromone of the Rice moth (scientific name: *Corcyra cephalonica*), 6,10,14-trimethyl-2-pentadecanol (Non-Patent Literature 1 listed below).

The following process for preparing a 1-arenesulfonyloxy-5-haloalkane compound has been reported. A process for preparing, for example, the 1-arenesulfonyloxy-5-haloalkane compound, 1-bromo-3-methyl-5-(*p-*toluenesulfonyloxy)pentane includes subjecting 4-methyltetrahydropyran to a ring-opening reaction with acetyl bromide in the presence of a zinc chloride catalyst to obtain 1-acetoxy-5-bromo-3-methylpentane with a yield of 89%, then eliminating an acetyl group of the 1-acetoxy-5-bromo-3-methylpentane thus obtained with water in a methanol solvent in the presence of a *p*-toluenesulfonic acid catalyst to form 5-bromo-3-methyl-1-pentanol with a yield of 82% (Non-Patent Literature 2 listed below). A hydroxy group of the 5-bromo-3-methyl-1-pentanol thus obtained is then tosylated with p-toluenesulfonyl chloride in a pyridine solvent to form a 1-arenesulfonyloxy-5-haloalkane compound with a yield of 75% (Non-Patent Literature 3 listed below).

Another reported process for preparing the 1-arenesulfonyloxy-5-haloalkane compound, 1-chloro-5-(*p*-toluenesulfonyloxy)pentane, includes, for example, tosylating a hydroxy group of 5-chloro-1-pentanol with p-toluenesulfonyl chloride in a pyridine solvent in the presence of a 4-dimethylaminopyridine (DMAP) catalyst with a yield of 96% (Non-Patent Literature 4 listed below).

### PRIOR ART

### [Non-Patent Literatures]

[Non-Patent Literature 1] D. R. Hall et al., J. Chem. Ecol., 1987, 13 (7), 1575-1589.
[Non-Patent Literature 2] V. N. Odinokov et al., Chemistry of Natural Compounds, 1989, 25, 236-239.
[Non-Patent Literature 3] V. N. Odinokov et al., Chemistry of Natural Compounds, 1990, 26, 86-87.
[Non-Patent Literature 4] K. Mori et al., Tetrahedron, 2010, 66 (14), 2642-2653.
[Non-Patent Literature 5] Y. Ishii et al., Journal of the Society of Chemical Industry, Japan, 1966, 69 (7), 1388-1393.

### OBJECT OF THE INVENTION

However, the processes for preparing 1-arenesulfonyloxy-5-haloalkane compounds according to Non-Patent Literatures 2 and 3 require the three steps of a ring-opening reaction, elimination of an acetyl group, and arenesulfonyloxylation (tosylation), and so the number of steps is high, and the three-step yield is as low as 55%. Moreover, the use of large amounts of solvents in the elimination and tosylation steps increases the environmental impact, and the preparation process is unsuited to industrialization.

The process for preparing the 1-arenesulfonyloxy-5-haloalkane compound according to Non-Patent Literature 4 also is unsuited to industrialization because the starting material, 5-chloro-1-pentanol, is extremely expensive. Furthermore, the aforesaid preparation process is not preferable in view of green chemistry because pyridine is used as a base in solvent amounts (large amounts).

The present invention was made in view of the aforementioned circumstances, and aims to provide a process for preparing a 1-arenesulfonyloxy-5-haloalkane compound efficiently from the inexpensive raw material, tetrahydropyran compound, in one step. The present invention also aims to provide the synthetic intermediate, 1-arenesulfonyloxy-5-halo-3-methylpentane compound, which is useful for synthesizing insect pheromone compounds, and to provide a process for preparing the sex pheromone compound, 6,10,14-trimethyl-2-pentadecanol.

### SUMMARY OF THE INVENTION

As a result of intensive research to overcome the aforesaid problems of the prior art, the present inventors found that a 1-arenesulfonyloxy-5-haloalkane compound may be efficiently prepared in one step by subjecting a tetrahydropyran compound having a six-member ring skeleton to a ring-opening reaction with an arenesulfonyl halide compound. Because it has been reported in Non-Patent Literature 5 that tetrahydropyran compounds having six-member ring skeletons are 100 times less reactive than tetrahydrofuran compounds having five-member ring skeletons in ring-opening reactions, it was surprising that the aforesaid problem could be overcome.

It was also found that a 1-arenesulfonyloxy-5-haloalkane compound may be used in a subsequent step without filtering or purification because the addition of methanol after the ring-opening reaction resulted in extremely good phase separation.

According to a first aspect of the present invention, there is provided a process for preparing a 1-arenesulfonyloxy-5-haloalkane compound of the following general formula (3): 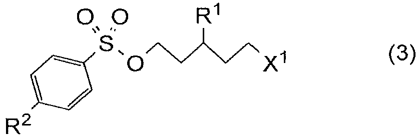
wherein R¹ and R² represent, independently of each other, a hydrogen atom or a methyl group, and X¹ represents a halogen atom,
the process comprising a step of:
   subjecting a tetrahydropyran compound of the following general formula (1): 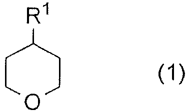
wherein R¹ represents a hydrogen atom or a methyl group,
to a ring-opening reaction with an arenesulfonyl halide compound of the following general formula (2): 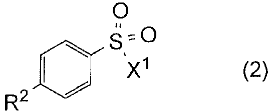
wherein R² represents a hydrogen atom or a methyl group, and X¹ represents a halogen atom,
in the presence of zinc chloride to form the aforesaid 1-arenesulfonyloxy-5-haloalkane compound (3).

According to a second aspect of the present invention, there is provided a process for preparing a 1-halo-3,7,11-trimethyldodecane compound of the following general formula (5): 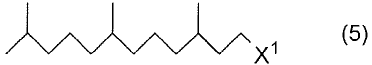
wherein X¹ represents a halogen atom,
the process comprising steps of:
   the aforesaid process of the first aspect for preparing a 1-arenesulfonyloxy-5-haloalkane compound (3) wherein R¹ = CH₃ (hereinafter, referred to as a 1-arenesulfonyloxy-5-haloalkane compound (3: R¹ = CH₃)) from a tetrahydropyran compound (1) wherein R¹ = CH₃ (hereinafter, referred to as a tetrahydropyran compound (1: R¹ = CH₃)), and
   subjecting a nucleophilic reagent, 2,6-dimethylheptyl compound, of the following general formula (4): 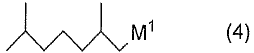
wherein M¹ represents Li, MgZ¹, CuZ¹, or CuLiZ¹, and Z¹ represents a halogen atom or a 2,6-dimethylheptyl group,
to a coupling reaction with the 1-arenesulfonyloxy-5-halo-3-methylpentane compound (3: R¹ = CH₃)
to form the aforesaid 1-halo-3,7,11-trimethyldodecane compound (5).

According to a third aspect of the present invention, there is provided a process for preparing 6,10,14-trimethyl-2-pentadecanol of the following formula (8): 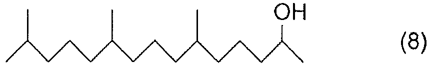
the process comprising steps of:
   the aforesaid process of the first aspect for preparing the aforesaid 1-arenesulfonyloxy-5-haloalkane compound (3) wherein R¹ = CH₃ (hereinafter, referred to as the 1-arenesulfonyloxy-5-haloalkane compound (3: R¹ = CH₃)) from a tetrahydropyran compound (1) wherein R¹ = CH₃ (hereinafter, referred to as the tetrahydropyran compound (1: R¹ = CH₃)),
   subjecting a nucleophilic reagent, 2,6-dimethylheptyl compound, of the following general formula (4): 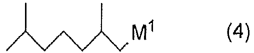
wherein M¹ represents Li, MgZ¹, CuZ¹, or CuLiZ¹, and Z¹ represents a halogen atom or a 2,6-dimethylheptyl group,
to a coupling reaction with the 1-arenesulfonyloxy-5-halo-3-methylpentane compound (3: R¹ = CH₃) to form a 1-halo-3,7,11-trimethyldodecane compound of the following general formula (5): 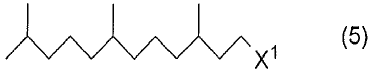
wherein X¹ represents a halogen atom,
   converting the 1-halo-3,7,11-trimethyldodecane compound (5) thus obtained into a nucleophilic reagent, 3,7,11-trimethyldodecyl compound, of the following general formula (6): 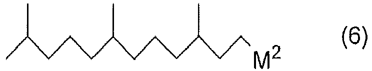
wherein M² represents Li, MgZ², CuZ², or CuLiZ², and Z² represents a halogen atom or a 3,7,11-trimethyldodecyl group,
   and
   subjecting the nucleophilic reagent, 3,7,11-trimethyldodecyl compound (6) thus obtained , to a nucleophilic addition reaction with propylene oxide of the following formula (7): 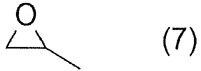
to form the aforesaid 6,10,14-trimethyl-2-pentadecanol (8).

According to the present invention, the target compound, 1-arenesulfonyloxy-5-haloalkane compound, may be efficiently prepared in fewer steps with a low environmental impact and a high yield. According to the present invention, the target compound, 1-arenesulfonyloxy-5-haloalkane compound, may be economically prepared. According to the present invention, purification by column chromatography and/or distillation is unnecessary, making possible the removal of impurities by phase separation, which may be scaled up. According to the present invention, among 1-arenesulfonyloxy-5-haloalkane compounds, a 1-arenesulfonyloxy-5-halo-3-methylpentane compound wherein R¹ = CH₃ may be used to prepare the aforesaid 1-arenesulfonyloxy-5-haloalkane compound and the aforesaid 6,10,14-trimethyl-2-pentadecanol.

### DETAILED DESCRIPTION OF THE INVENTION

### (I) Process for preparing 1-arenesulfonyloxy-5-haloalkane compound of the following general formula (3)

### (i) A 1-arenesulfonyloxy-5-haloalkane compound (3) is represented by the following general formula (3):

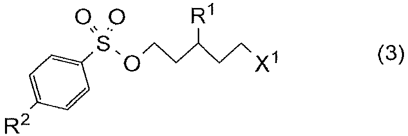

In the general formula (3) above, R¹ and R² represent, independently of each other, a hydrogen atom or a methyl group, and X¹ represents a halogen atom. Specific examples of the halogen atom X¹ include a chlorine atom, a bromine atom, and an iodine atom. The halogen atom X¹ is preferably a chlorine atom and a bromine atom. By using said chlorine atom and bromine atom, a preferred reactivity may be ensured. The halogen atom X¹ is more preferably a chlorine atom. By using said chlorine atom, a more preferred reactivity may be ensured.

### (ii) A process for preparing the aforesaid 1-arenesulfonyloxy-5-haloalkane compound (3) will be explained in detail below.

The 1-arenesulfonyloxy-5-haloalkane compound (3) may be prepared by subjecting a tetrahydropyran compound of the following general formula (1) to a ring-opening reaction with an arenesulfonyl halide compound of the following general formula (2) in the presence of zinc chloride, as shown by the following chemical reaction formula:

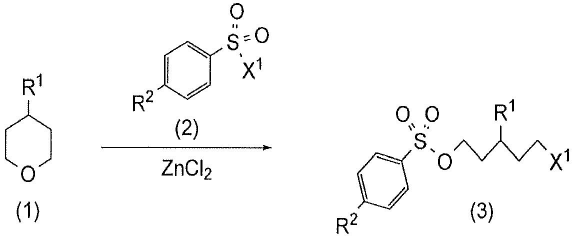

First, the tetrahydropyran compound (1) will be explained in detail below.

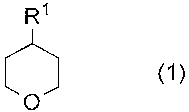

In the general formula (1) above, R¹ represents a hydrogen atom or a methyl group.

Specific examples of the tetrahydropyran compound (1) include tetrahydropyran (R¹ = H) and 4-methyltetrahydropyran (R¹ = CH₃). The tetrahydropyran compound (1) is preferably 4-methyltetrahydropyran. By using said 4-methyltetrahydropyran, a preferred preparation of an insect pheromone having a methyl branch may be ensured.

The tetrahydropyran compound (1) may be a commercially available one.

The arenesulfonyl halide compound (2) will be explained in detail below.

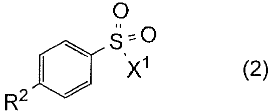

In the arenesulfonyl halide compound (2), R² represents a hydrogen atom or a methyl group, and X¹ represents a halogen atom. Specific examples of the halogen atom X¹ include a chlorine atom, a bromine atom, and an iodine atom. The halogen atom X¹ is preferably a chlorine atom and a bromine atom. By using said chlorine atom and bromine atom, a preferred reactivity may be ensured. The halogen atom X¹ is more preferably a chlorine atom. By using said chlorine atom, a more preferred reactivity may be ensured.

Specific examples of the arenesulfonyl halide compound (2) include benzenesulfonyl halide compounds (R² = H) such as benzenesulfonyl chloride, benzenesulfonyl bromide, and benzenesulfonyl iodide; and toluenesulfonyl halide compounds (R² = CH₃) such as toluenesulfonyl chloride, toluenesulfonyl bromide, and toluenesulfonyl iodide. The arenesulfonyl halide compound (2) is preferably benzenesulfonyl chloride, benzenesulfonyl bromide, toluenesulfonyl chloride, and toluenesulfonyl bromide. By using said benzenesulfonyl chloride, benzenesulfonyl bromide, toluenesulfonyl chloride, and toluenesulfonyl bromide, preferred economy and/or handling may be ensured. The arenesulfonyl halide compound (2) is more preferably benzenesulfonyl chloride and toluenesulfonyl chloride. By using said benzenesulfonyl chloride and toluenesulfonyl chloride, more preferred economy and/or handling may be ensured. The arenesulfonyl halide compound (2) is even more preferably benzenesulfonyl chloride. By using said benzenesulfonyl chloride, even more preferred economy and/or handling may be ensured.

The arenesulfonyl halide compound (2) may be used alone or in combination thereof, if necessary. The arenesulfonyl halide compound (2) may be a commercially available one.

The amount of the arenesulfonyl halide compound (2) used, per mol of the tetrahydropyran compound (1), is preferably 0.5 to 2.0 mol, more preferably 0.7 to 1.5 mol, and even more preferably 0.8 to 1.1 mol. By using said preferred amount, said more preferred amount, and said even more preferred amount, a preferred reactivity, a more preferred reactivity, and an even more preferred reactivity may be ensured. If 1.3 equivalents or more of the tetrahydropyran compound (1), per the arenesulfonyl halide compound (2), are used in the ring-opening reaction, the reaction rate decreases. If 1.5 equivalents or more of the tetrahydropyran compound (1), per the arenesulfonyl halide compound (2), are used in the ring-opening reaction, the reaction rate decreases further. If 2.0 equivalents or more are used, the reaction becomes extremely slow, and the reaction no longer goes to completion.

A solvent may be incorporated in the ring-opening reaction, if necessary.

Examples of the solvent include general solvents such as, for example, hydrocarbon solvents such as hexane, heptane, benzene, toluene, xylene, and cumene. The solvent is preferably toluene, xylene, and cumene. By using said toluene, xylene, and cumene, a preferred reactivity may be ensured. The solvent is more preferably toluene and xylene. By using said toluene and xylene, a more preferred reactivity may be ensured.

The solvent may be used alone or in combination thereof, if necessary. The solvent may be a commercially available one.

When a solvent is incorporated in the ring-opening reaction, the amount of the solvent used, per mol of the tetrahydropyran compound (1), is preferably more than 0 up to 3,000 g, and more preferably more than 0 up to 800 g.

By incorporating the solvent, the reaction rate is greatly reduced, and so it is desirable to carry out the reaction solvent-less without incorporating the aforesaid solvent.

The reaction temperature of the ring-opening reaction is preferably 60 to 180°C, more preferably 70 to 120°C, and even more preferably 75 to 100°C. By using said preferred reaction temperature, said more preferred reaction temperature, and said even more preferred reaction temperature, a preferred reactivity, a more preferred reactivity, and an even more preferred reactivity may be ensured.

The reaction time of the ring-opening reaction varies, depending on the production scale, and is preferably 0.1 to 100 hours, more preferably 0.3 to 50 hours, and even more preferably 0.5 to 20 hours. By using said preferred reaction time, said more preferred reaction time, and said even more preferred reaction time, a preferred reactivity, a more preferred reactivity, and an even more preferred reactivity may be ensured.

Although the 1-arenesulfonyloxy-5-haloalkane compound (3) may be prepared as explained above, if brine or the like is added after quenching the reaction with an aqueous solution containing water, the separation between the aqueous layer and the organic layer is extremely poor, and a boundary layer appears between the aqueous layer and the organic layer where the aqueous layer and the organic layer are mixed. The boundary layer remains even after adding an extraction solvent, adding sodium chloride, and/or heating. Therefore, a clean organic layer may be obtained by removing the boundary layer when phase-separating, but the yield will be reduced by loss of the organic layer. If, on the other hand, the boundary layer is included in the organic layer side, a large amount of salts precipitate after concentration, and so the reaction mixture cannot be used as such in a reaction of a subsequent step, and it is necessary to carry out a filtration step or a purification step by silica gel column chromatography or distillation. However, by adding an alcohol compound (9) after quenching the reaction with an aqueous solution, separation of the organic layer and the aqueous layer is extremely good, and so the addition of the alcohol compound (9) is preferred. Examples of the alcohol compound (9) include methanol, ethanol, n-propanol, isopropyl alcohol, n-butanol, 2-butanol, n-pentanol, ethylene glycol, and propylene glycol. The alcohol compound (9) is preferably methanol, ethanol, n-propanol, and isopropyl alcohol. By using said methanol, ethanol, n-propanol, and isopropyl alcohol, a preferred separation of the organic layer and the aqueous layer may be ensured. The alcohol compound (9) is more preferably methanol and ethanol. By using said methanol and ethanol, a more preferred separation of the organic layer and the aqueous layer may be ensured. The alcohol compound (9) is even more preferably methanol. By using said methanol, an even more preferred separation of the organic layer and the aqueous layer may be ensured.

The amount of the alcohol compound (9) added, per mol of the tetrahydropyran compound (1), is preferably more than 0 up to 1,000 g, more preferably 15 g to 500 g, even more preferably 30 g to 300 g, and particularly preferably 50 g to 150 g.

Thus, by adding the alcohol compound (9) in the post-treatment, separation of the organic layer and the aqueous layer is extremely good, and a boundary layer does not appear. As a result, the product, 1-arenesulfonyloxy-5-haloalkane compound (3), may be incorporated in a reaction of a subsequent step without a filtration step and/or a purification step because there is no loss of the organic layer due to phase separation and no precipitation of salts after phase separation and concentration.

In the 1-arenesulfonyloxy-5-haloalkane compound (3) thus prepared, R¹ and R² represent a hydrogen atom or a methyl group, and the X¹ represents a halogen atom. Here, depending on the combination of R¹ of the 1-arenesulfonyloxy-5-haloalkane compound (3) and R² of the arenesulfonyl halide compound (2) used in the ring-opening reaction, R¹ and R² each may be a hydrogen atom, R¹ and R² each may be a methyl group, R¹ and R² may be respectively a hydrogen atom and a methyl group, or R¹ and R² may be respectively a methyl group and a hydrogen atom.

Specific examples of the 1-arenesulfonyloxy-5-haloalkane compound (3) include the following compounds:
1-benzenesulfonyloxy-5-halopentane compounds such as 1-benzenesulfonyloxy-5-chloropentane, 1-benzenesulfonyloxy-5-bromopentane, and 1-benzenesulfonyloxy-5-iodopentane;
1-halo-5-(p-toluenesulfonyloxy)pentane compounds such as 1-chloro-5-(p-toluenesulfonyloxy)pentane, 1-bromo-5-(p-toluenesulfonyloxy)pentane, and 1-iodo-5-(p-toluenesulfonyloxy)pentane;
1-benzenesulfonyloxy-5-halo-3-methylpentane compounds such as 1-benzenesulfonyloxy-5-chloro-3-methylpentane, 1-benzenesulfonyloxy-5-bromo-3-methylpentane, and 1-benzenesulfonyloxy-5-iodo-3-methylpentane; and
1-halo-3-methyl-5-(*p*-toluenesulfonyloxy)pentane compounds such as 1-chloro-3-methyl-5-(p-toluenesulfonyloxy)pentane, 1-bromo-3-methyl-5-(p-toluenesulfonyloxy)pentane, and 1-iodo-3-methyl-5-(*p*-toluenesulfonyloxy)pentane.

The 1-arenesulfonyloxy-5-haloalkane compound (3) is preferably 1-benzenesulfonyloxy-5-chloropentane, 1-benzenesulfonyloxy-5-bromopentane, 1-chloro-5-(*p**-***toluenesulfonyloxy)pentane, 1-bromo-5-(p-toluenesulfonyloxy)pentane, 1-benzenesulfonyloxy-5-chloro-3-methylpentane, 1-benzenesulfonyloxy-5-bromo-3-methylpentane, 1-chloro-3-methyl-5-(*p*-toluenesulfonyloxy)pentane, and 1-bromo-3-methyl-5-(p-toluenesulfonyloxy)pentane. By using said 1-benzenesulfonyloxy-5-chloropentane, 1-benzenesulfonyloxy-5-bromopentane, 1-chloro-5-(p-toluenesulfonyloxy)pentane, 1-bromo-5-(*p*-toluenesulfonyloxy)pentane, 1-benzenesulfonyloxy-5-chloro-3-methylpentane, 1-benzenesulfonyloxy-5-bromo-3-methylpentane, 1-chloro-3-methyl-5-(*p*-toluenesulfonyloxy)pentane, and 1-bromo-3-methyl-5-(*p*-toluenesulfonyloxy)pentane, a preferred reactivity may be ensured. The 1-arenesulfonyloxy-5-haloalkane compound (3) is more preferably 1-benzenesulfonyloxy-5-chloropentane, 1-chloro-5-(*p*-toluenesulfonyloxy)pentane, 1-benzenesulfonyloxy-5-chloro-3-methylpentane, and 1-chloro-3-methyl-5-(*p*-toluenesulfonyloxy)pentane. By using said 1-benzenesulfonyloxy-5-chloropentane, 1-chloro-5-(*p-*toluenesulfonyloxy)pentane, 1-benzenesulfonyloxy-5-chloro-3-methylpentane, and 1-chloro-3-methyl-5-(*p*-toluenesulfonyloxy)pentane, a more preferred reactivity may be ensured. The 1-arenesulfonyloxy-5-haloalkane compound (3) is even more preferably 1-benzenesulfonyloxy-5-chloro-3-methylpentane and 1-benzenesulfonyloxy-5-chloropentane. By using said 1-benzenesulfonyloxy-5-chloro-3-methylpentane and 1-benzenesulfonyloxy-5-chloropentane, an even more preferred handling may be ensured. The 1-arenesulfonyloxy-5-haloalkane compound (3) is particularly preferably 1-benzenesulfonyloxy-5-chloro-3-methylpentane. By using said 1-benzenesulfonyloxy-5-chloro-3-methylpentane, a particularly preferred handling may be ensured.

### (II) Process for preparing 1-halo-3,7,11-trimethyldodecane compound (5) from 1-arenesulfonyloxy-5-haloalkane compound (3) when R¹ = CH₃, i.e., 1-arenesulfonyloxy-5-halo-3-methylpentane compound (3) (hereinafter, referred to as 1-arenesulfonyloxy-5-haloalkane compound (3: R¹ = CH₃))

The 1-halo-3,7,11-trimethyldodecane compound (5) may be prepared by subjecting the aforesaid 1-arenesulfonyloxy-5-halo-3-methylpentane compound (3: R¹ = CH₃) to a coupling reaction with a nucleophilic reagent, 2,6-dimethylheptyl compound (4), as shown by the following chemical reaction formula: 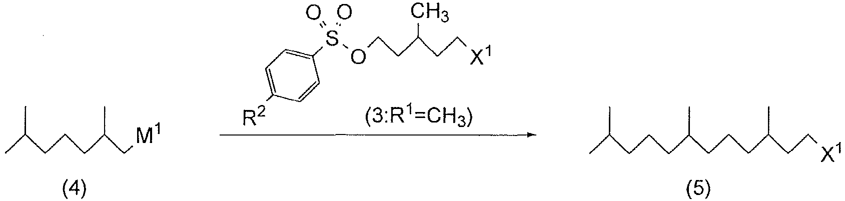
(a) First, the aforesaid nucleophilic reagent, 2,6-dimethylheptyl compound (4), will be explained in detail below. The nucleophilic reagent, 2,6-dimethylheptyl compound (4), is represented by the following general formula (4):

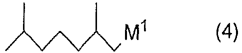

In the general formula (4) above, M¹ represents Li, MgZ¹, CuZ¹, or CuLiZ¹, and Z¹ represents a halogen atom or a 2,6-dimethylheptyl group. Specific examples of the halogen atom Z¹ include a chlorine atom, a bromine atom, and an iodine atom. The halogen atom Z¹ is preferably a chlorine atom and a bromine atom. By using said chlorine atom and bromine atom, a preferred reactivity may be ensured. The halogen atom Z¹ is particularly preferably a chlorine atom. By using said chlorine atom, a particularly preferred reactivity may be ensured.

Specific examples of the nucleophilic reagent, 2,6-dimethylheptyl compound (4), include the following compounds:
2,6-dimethylheptyllithium (when M¹ = Li);
2,6-dimethylheptylmagnesium halide compounds (when M¹ = MgZ¹) such as 2,6-dimethylheptylmagnesium chloride, 2,6-dimethylheptylmagnesium bromide, and 2,6-dimethylheptylmagnesium iodide (i.e., Grignard reagents);
bis(2,6-dimethylheptyl)cuprate compounds (when M¹ = CuZ¹) such as bis(2,6-dimethylheptyl)cuprate; and
lithium bis(2,6-dimethylheptyl)cuprate compounds (when M¹ = CuLiZ¹) (such as lithium bis(2,6-dimethylheptyl)cuprate) (i.e., Gilman reagents).

The nucleophilic reagent, 2,6-dimethylheptyl compound (4), is preferably Grignard reagents such as 2,6-dimethylheptylmagnesium halide compounds. By using said 2,6-dimethylheptylmagnesium halide compounds, a preferred reactivity may be ensured.

### (b) A process for preparing the aforesaid nucleophilic reagent, 2,6-dimethylheptyl compound (4), will be explained in detail below.

The nucleophilic reagent, 2,6-dimethylheptyl compound (4), may be prepared from, for example, a 1-halo-2,6-dimethylheptane compound of the following general formula (10):

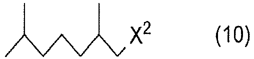

In the general formula (10) above, X² represents a halogen atom. Specific examples of the halogen atom X² include a chlorine atom, a bromine atom, and an iodine atom. The halogen atom X² is preferably a chlorine atom and a bromine atom. By using said chlorine atom and bromine atom, a preferred reactivity may be ensured. The halogen atom X² is more preferably a chlorine atom. By using said chlorine atom, a more preferred reactivity may be ensured.

Specific examples of the 1-halo-2,6-dimethylheptane compound (10) include 1-chloro-2,6-dimethylheptane, 1-bromo-2,6-dimethylheptane, and 1-iodo-2,6-dimethylheptane. The 1-halo-2,6-dimethylheptane compound (10) is preferably 1-chloro-2,6-dimethylheptane and 1-bromo-2,6-dimethylheptane. By using said 1-chloro-2,6-dimethylheptane and 1-bromo-2,6-dimethylheptane, a preferred reactivity may be ensured. The 1-halo-2,6-dimethylheptane compound (10) is more preferably 1-chloro-2,6-dimethylheptane. By using said 1-chloro-2,6-dimethylheptane, a more preferred reactivity may be ensured.

A process for preparing, for example, a nucleophilic reagent, 2,6-dimethylheptyl compound (4), when M¹ is MgZ¹, i.e., a 2,6-dimethylheptylmagnesium halide reagent (4: M¹ = MgZ¹) (i.e., a Grignard reagent) will be described in detail as an example.

The Grignard reagent, 2,6-dimethylheptylmagnesium halide reagent (4: M¹ = MgZ¹), may be prepared by, for example, reacting the aforesaid 1-halo-2,6-dimethylheptane compound (10) with magnesium in a solvent, as shown by the following chemical reaction formula:

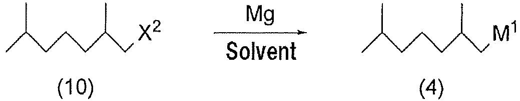

The amount of magnesium used when preparing the 2,6-dimethylheptylmagnesium halide reagent (4: M¹ = MgZ¹), per mol of the 1-halo-2,6-dimethylheptane compound (10), is preferably 1.0 to 2.0 gram atoms. By using said preferred amount, a preferred completion of the reaction may be ensured.

Examples of the solvent include general solvents such as, for example, ether solvents such as tetrahydrofuran (THF), 2-methyltetrahydrofuran (2-MeTHF), diethyl ether, dibutyl ether, 4-methyltetrahydropyran (MTHP), cyclopentylmethylether, and 1,4-dioxane; and hydrocarbon solvents such as hexane, heptane, benzene, toluene, xylene, and cumene. The solvent is preferably hydrocarbon solvents such as toluene and xylene; and ether solvents such as tetrahydrofuran, 2-methyltetrahydrofuran, and 4-methyltetrahydropyran. By using said hydrocarbon solvents such as toluene and xylene; and ether solvents such as tetrahydrofuran, 2-methyltetrahydrofuran, and 4-methyltetrahydropyran, a preferred reaction rate of production of the aforesaid Grignard reagent may be ensured. The solvent is more preferably tetrahydrofuran, 2-methyltetrahydrofuran, and 4-methyltetrahydropyran. By using said tetrahydrofuran, 2-methyltetrahydrofuran, and 4-methyltetrahydropyran, a more preferred reaction rate of production of the aforesaid Grignard reagent may be ensured.

The solvent may be used alone or in combination thereof, if necessary. The solvent may be a commercially available one.

The amount of the solvent used, per mol of the 1-halo-2,6-dimethylheptane compound (10), is preferably 30 to 5,000 g, more preferably 50 to 3,000 g, and even more preferably 100 to 1,000 g. By using said preferred amount, said more preferred amount, and said even more preferred amount, a preferred reactivity, a more preferred reactivity, and an even more preferred reactivity may be ensured.

The reaction temperature varies, depending on the solvent used, and is preferably 30 to 120°C, more preferably 50 to 100°C, and even more preferably 60 to 80°C. By using said preferred reaction temperature, said more preferred reaction temperature, and said even more preferred reaction temperature, a preferred reactivity, a more preferred reactivity, and an even more preferred reactivity may be ensured.

The reaction time varies, depending on the solvent used and/or the production scale, and is preferably 0.5 to 100 hours. By using said preferred reaction time, a preferred reactivity may be ensured.

(c) A coupling reaction of a nucleophilic reagent, 2,6-dimethylheptyl compound (4), with a 1-arenesulfonyloxy-5-halo-3-methylpentane compound (3: R¹ = CH₃) will be explained in detail below.

The amount of the 1-arenesulfonyloxy-5-halo-3-methylpentane compound (3: R¹ = CH₃) used in the coupling reaction, per mol of the nucleophilic reagent, 2,6-dimethylheptyl compound (4), is preferably 0.7 to 2.0 mol, more preferably 0.8 to 1.7 mol, and even more preferably 0.9 to 1.4 mol. By using said preferred amount, said more preferred amount, and said even more preferred amount, preferred economy, more preferred economy, and even more preferred economy may be ensured.

A solvent may be incorporated in the coupling reaction, if necessary. Examples of the solvent include general solvents such as, for example, ether solvents such as tetrahydrofuran (THF), 2-methyltetrahydrofuran (2-MeTHF), diethyl ether, dibutyl ether, 4-methyltetrahydropyran (MTHP), cyclopentylmethylether, and 1,4-dioxane; hydrocarbon solvents such as hexane, heptane, benzene, toluene, xylene, and cumene; and polar solvents such as N,N-dimethylformamide (DMF), N,N-dimethylacetamide (DMAC), *N*-methylpyrrolidone (NMP), dimethyl sulfoxide (DMSO), γ-butyrolactone (GBL), acetonitrile, N,N'-dimethylpropylene urea (DMPU), hexamethylphosphoric triamide (HMPA), dichloromethane, and chloroform. The solvent is preferably hydrocarbon solvents such as toluene and xylene; and ether solvents such as tetrahydrofuran, 2-methyltetrahydrofuran, and 4-methyltetrahydropyran. By using said hydrocarbon solvents such as toluene and xylene; and ether solvents such as tetrahydrofuran, 2-methyltetrahydrofuran, and 4-methyltetrahydropyran, a preferred reactivity may be ensured. The solvent is more preferably tetrahydrofuran, 2-methyltetrahydrofuran, and 4-methyltetrahydropyran. By using said tetrahydrofuran, 2-methyltetrahydrofuran, and 4-methyltetrahydropyran, a more preferred reactivity may be ensured.

The solvent may be used alone or in combination thereof, if necessary. The solvent may be a commercially available one.

The amount of the solvent used, per mol of the nucleophilic reagent, 2,6-dimethylheptyl compound (4), is preferably 30 to 5,000 g, more preferably 50 to 3,000 g, and even more preferably 100 to 1,000 g. By using said preferred amount, said more preferred amount, and said even more preferred amount, a preferred reactivity, a more preferred reactivity, and an even more preferred reactivity may be ensured.

The coupling reaction of the nucleophilic reagent, 2,6-dimethylheptyl compound (4), with the 1-arenesulfonyloxy-5-halo-3-methylpentane compound (3: R¹ = CH₃) may be carried out in the presence of a catalyst, if necessary.

Examples of the catalyst include copper compounds such as cuprous halides such as cuprous chloride, cuprous bromide, and cuprous iodide, and cupric halides such as cupric chloride, cupric bromide, and cupric iodide; iron compounds such as iron(II) chloride, iron(III) chloride, iron(II) bromide, iron(III) bromide, iron(II) iodide, iron(III) iodide, and iron(III)acetylacetonate; silver compounds such as silver chloride, silver nitrate, and silver acetate; titanium compounds such as titanium tetrachloride, titanium tetrabromide, titanium(IV) methoxide, titanium(IV) ethoxide, titanium(IV) isopropoxide, and titanium(IV) oxide; palladium(II) compounds such as dichlorobis(triphenylphosphine)palladium and dichloro[1,1'-bis(diphenylphosphino)ferrocene]palladium; and nickel compounds such as nickel chloride, dichloro[1,2-bis(diphenylphosphino)ethane]nickel(II), and dichlorobis(triphenylphosphine)nickel(II). The catalyst is preferably a copper compound. By using said copper compound, a preferred reactivity and/or economy may be ensured. The catalyst is more preferably cupric halides such as cupric chloride, cupric bromide, and cupric iodide. By using said cupric halides such as cupric chloride, cupric bromide, and cupric iodide, a more preferred reactivity and/or economy may be ensured.

The catalyst may be used alone or in combination thereof, if necessary. The catalyst may be a commercially available one.

The amount of the catalyst used, per mol of the 1-halo-2,6-dimethylheptane compound (10), is preferably 0.0001 to 1.0 mol, more preferably 0.0005 to 0.5 mol, and even more preferably 0.001 to 0.3 mol. By using said preferred amount, said more preferred amount, and said even more preferred amount, a preferred reaction rate and/or post-treatment, a more preferred reaction rate and/or post-treatment, and an even more preferred reaction rate and/or post-treatment may be ensured.

When the coupling reaction is carried out in the presence of a catalyst, a co-catalyst may be used, if necessary. Examples of the co-catalyst include trialkyl phosphite compounds having 3 to 9 carbon atoms such as triethyl phosphite; and arylphosphine compounds having 18 to 44 carbon atoms such as triphenylphosphine, tritolylphosphine, and 2,2'-bis(diphenylphosphino)-1,1'-binaphthyl (BINAP). The co-catalyst is preferably trialkyl phosphite. By using said trialkyl phosphite, a preferred reactivity may be ensured. The co-catalyst is more preferably triethyl phosphite. By using said triethyl phosphite, a more preferred reactivity may be ensured.

The co-catalyst may be used alone or in combination thereof, if necessary. The co-catalyst may be a commercially available one.

The amount of the co-catalyst used, per mol of the 1-halo-2,6-dimethylheptane compound (10), is preferably 0.0001 to 1.0 mol, more preferably 0.0005 to 0.5 mol, and even more preferably 0.001 to 0.3 mol.

When the coupling reaction is carried out in the presence of a catalyst, a lithium salt may be incorporated, if necessary. The lithium salt may be incorporated in the coupling reaction with the aforesaid co-catalyst, or may be incorporated alone without using the aforesaid co-catalyst. Examples of the lithium salt include lithium halides such as lithium chloride, lithium bromide, and lithium iodide; lithium nitrate; and lithium carbonate. The lithium salt is preferably lithium halides such as lithium chloride; and lithium nitrate. By using said lithium halides such as lithium chloride; and lithium nitrate, a preferred reactivity may be ensured.

The lithium salt may be used alone or in combination thereof, if necessary. The lithium salt may be a commercially available one.

The amount of the lithium salt used in the coupling reaction, per mol of the 1-halo-2,6-dimethylheptane compound (10), is preferably 0.0001 to 1.0 mol, more preferably 0.0005 to 0.5 mol, and even more preferably 0.001 to 0.3 mol. By using said preferred amount, said more preferred amount, and said even more preferred amount, a preferred reactivity, a more preferred reactivity, and an even more preferred reactivity may be ensured.

The reaction temperature of the coupling reaction varies, depending on the nucleophilic reagent, 2,6-dimethylheptyl compound (4), used, and is preferably -78 to 100°C, more preferably -25 to 70°C, even more preferably -10 to 50°C, and particularly preferably 10 to 35°C. By using said preferred reaction temperature, said more preferred reaction temperature, said even more preferred reaction temperature, and said particularly preferred reaction temperature, a preferred reactivity, a more preferred reactivity, an even more preferred reactivity, and a particularly preferred reactivity may be ensured.

The reaction time of the coupling reaction varies, depending on the solvent used and/or the production scale, and is preferably 0.5 to 100 hours. By using said preferred reaction time, a preferred reactivity may be ensured.

### (III) A process for preparing 6,10,14-trimethyl-2-pentadecanol (8) will be explained in detail below.

6,10,14-Trimethyl-2-pentadecanol (8) may be prepared by converting a 1-halo-3,7,11-trimethyldodecane compound (5) into a nucleophilic reagent, 3,7,11-trimethyldodecyl compound (6), and then subjecting the nucleophilic reagent, 3,7,11-trimethyldodecyl compound (6), to a nucleophilic addition reaction with propylene oxide (7) as shown by, for example, the following chemical reaction formula: 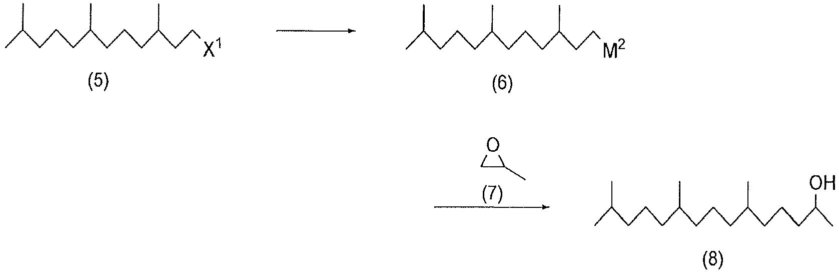
(a) First, the aforesaid nucleophilic reagent, 3,7,11-trimethyldodecyl compound (6), will be explained in detail below. The nucleophilic reagent, 3,7,11-trimethyldodecyl compound (6), is represented by the following general formula (6):

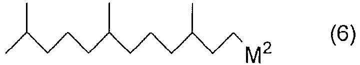

In the general formula (6) above, M² represents Li, MgZ², CuZ², or CuLiZ², and Z² represents a halogen atom or a 3,7,11-trimethyldodecyl group. Specific examples of the halogen atom Z² include a chlorine atom, a bromine atom, and an iodine atom. The halogen atom Z² is preferably a chlorine atom and a bromine atom. By using said chlorine atom and bromine atom, a preferred reactivity may be ensured. The halogen atom Z² is particularly preferably a chlorine atom. By using said chlorine atom, a particularly preferred reactivity may be ensured.

Specific examples of the nucleophilic reagent, 3,7,11-trimethyldodecyl compound (6), include the following compounds:
3,7,11-trimethyldodecyllithium (when M² = Li);
3,7,11-trimethyldodecylmagnesium halide compounds (when M² = MgZ²) such as 3,7,11-trimethyldodecylmagnesium chloride, 3,7,11-trimethyldodecylmagnesium bromide, and 3,7,11-trimethyldodecylmagnesium iodide (i.e., Grignard reagents);
bis(3,7,11-trimethyldodecyl)cuprate compounds (when M² = CuZ²) such as bis(3,7,11-trimethyldodecyl)cuprate; and
lithium bis(3,7,11-trimethyldodecyl)cuprate compounds (when M² = CuLiZ²) such as lithium bis(3,7,11-trimethyldodecyl)cuprate (i.e., Gilman reagents).

The nucleophilic reagent, 3,7,11-trimethyldodecyl compound (6), is preferably Grignard reagents such as 3,7,11-trimethyldodecylmagnesium halide compounds. By using said Grignard reagents such as 3,7,11-trimethyldodecylmagnesium halide compounds, a preferred reactivity may be ensured. The nucleophilic reagent, 3,7,11-trimethyldodecyl compound (6), is more preferably 3,7,11-trimethyldodecylmagnesium chloride and 3,7,11-trimethyldodecylmagnesium bromide. By using said 3,7,11-trimethyldodecylmagnesium chloride and 3,7,11-trimethyldodecylmagnesium bromide, a more preferred reactivity may be ensured. The nucleophilic reagent, 3,7,11-trimethyldodecyl compound (6), is even more preferably 3,7,11-trimethyldodecylmagnesium chloride. By using said 3,7,11-trimethyldodecylmagnesium chloride, an even more preferred reactivity may be ensured.

(b) A process for preparing the aforesaid nucleophilic reagent, 3,7,11-trimethyldodecyl compound (6), will be explained in detail below.

The nucleophilic reagent, 3,7,11-trimethyldodecyl compound (6), may be prepared from the aforesaid 1-halo-3,7,11-trimethyldodecane compound (5).

A process for preparing, for example, a nucleophilic reagent, 3,7,11-trimethyldodecyl compound (6), when M² is MgZ², i.e., a 3,7,11-trimethyldodecylmagnesium halide reagent (6: M² = MgZ²) (i.e., a Grignard reagent) will be described in detail as an example.

The Grignard reagent, 3,7,11-trimethyldodecylmagnesium halide reagent (6: M² = MgZ²), may be prepared by, for example, reacting the aforesaid 1-halo-3,7,11-trimethyldodecane compound (5) with magnesium in a solvent, as shown by the following chemical reaction formula:

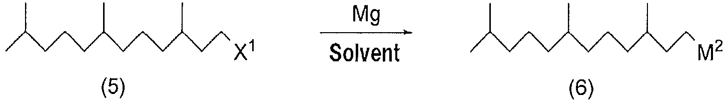

The amount of magnesium used when preparing the 3,7,11-trimethyldodecylmagnesium halide reagent (6), per mol of the 1-halo-3,7,11-trimethyldodecane compound (5), is preferably 1.0 to 2.0 gram atoms. By using said preferred amount, a preferred completion of the reaction may be ensured.

Examples of the solvent include general solvents such as, for example, ether solvents such as tetrahydrofuran (THF), 2-methyltetrahydrofuran (2-MeTHF), diethyl ether, dibutyl ether, 4-methyltetrahydropyran (MTHP), cyclopentylmethylether, and 1,4-dioxane; and hydrocarbon solvents such as hexane, heptane, benzene, toluene, xylene, and cumene. The solvent is preferably hydrocarbon solvents such as toluene and xylene; and ether solvents such as tetrahydrofuran, 2-methyltetrahydrofuran, and 4-methyltetrahydropyran. By using said hydrocarbon solvents such as toluene and xylene; and ether solvents such as tetrahydrofuran, 2-methyltetrahydrofuran, and 4-methyltetrahydropyran, a preferred reaction rate of production of the aforesaid Grignard reagent may be ensured. The solvent is more preferably tetrahydrofuran, 2-methyltetrahydrofuran, and 4-methyltetrahydropyran. By using said tetrahydrofuran, 2-methyltetrahydrofuran, and 4-methyltetrahydropyran, a more preferred reaction rate of production of the aforesaid Grignard reagent may be ensured.

The solvent may be used alone or in combination thereof, if necessary. The solvent may be a commercially available one.

The amount of the solvent used, per mol of the 1-halo-3,7,11-trimethyldodecane compound (5), is preferably 30 to 5,000 g, more preferably 50 to 3,000 g, and even more preferably 100 to 1,000 g. By using said preferred amount, said more preferred amount, and said even more preferred amount, a preferred reactivity, a more preferred reactivity, and an even more preferred reactivity may be ensured.

The reaction temperature varies, depending on the solvent used, and is preferably 30 to 120°C, more preferably 50 to 100°C, and even more preferably 60 to 80°C. By using said preferred reaction temperature, said more preferred reaction temperature, and said even more preferred reaction temperature, a preferred reactivity, a more preferred reactivity, and an even more preferred reactivity may be ensured.

The reaction time varies, depending on the solvent used and/or the production scale, and is preferably 0.5 to 100 hours. By using said preferred reaction time, a preferred reactivity may be ensured.

(c) A nucleophilic addition reaction of a nucleophilic reagent, 3,7,11-trimethyldodecyl compound (6), and propylene oxide (7) will be explained in detail below.

The amount of propylene oxide (7) used in the nucleophilic addition reaction, per mol of the nucleophilic reagent, 3,7,11-trimethyldodecyl compound (6), is preferably 0.8 to 2.0 mol, more preferably 0.9 to 1.7 mol, and even more preferably 1.0 to 1.5 mol. By using said preferred amount, said more preferred amount, and said even more preferred amount, preferred economy, more preferred economy, and even more preferred economy may be ensured.

A solvent may be incorporated in the nucleophilic addition reaction, if necessary. Examples of the solvent include general solvents such as, for example, ether solvents such as tetrahydrofuran (THF), 2-methyltetrahydrofuran (2-MeTHF), diethyl ether, dibutyl ether, 4-methyltetrahydropyran (MTHP), cyclopentylmethylether, and 1,4-dioxane; hydrocarbon solvents such as hexane, heptane, benzene, toluene, xylene, and cumene; and polar solvents such as N,N-dimethylformamide (DMF), N,N-dimethylacetamide (DMAC), *N*-methylpyrrolidone (NMP), dimethyl sulfoxide (DMSO), γ-butyrolactone (GBL), acetonitrile, N,N'-dimethylpropylene urea (DMPU), hexamethylphosphoric triamide (HMPA), dichloromethane, and chloroform. The solvent is preferably hydrocarbon solvents such as toluene and xylene; and ether solvents such as tetrahydrofuran, 2-methyltetrahydrofuran, and 4-methyltetrahydropyran. By using said hydrocarbon solvents such as toluene and xylene; and ether solvents such as tetrahydrofuran, 2-methyltetrahydrofuran, and 4-methyltetrahydropyran, a preferred reactivity may be ensured. The solvent is more preferably tetrahydrofuran, 2-methyltetrahydrofuran, and 4-methyltetrahydropyran. By using said tetrahydrofuran, 2-methyltetrahydrofuran, and 4-methyltetrahydropyran, a more preferred reactivity may be ensured.

The solvent may be used alone or in combination thereof, if necessary. The solvent may be a commercially available one.

When a solvent is incorporated in the nucleophilic addition reaction, the amount of the solvent used, per mol of the nucleophilic reagent, 3,7,11-trimethyldodecyl compound (6), is preferably 30 to 5,000 g, more preferably 50 to 3,000 g, and even more preferably 100 to 1,000 g. By using said preferred amount, said more preferred amount, and said even more preferred amount, a preferred reactivity, a more preferred reactivity, and an even more preferred reactivity may be ensured.

The nucleophilic addition reaction may be carried out in the presence of a catalyst, if necessary.

Examples of the catalyst include copper compounds such as cuprous halides such as cuprous chloride, cuprous bromide, and cuprous iodide, and cupric halides such as cupric chloride, cupric bromide, and cupric iodide. The catalyst is preferably cuprous halides. By using said cuprous halides, a preferred reactivity may be ensured. The catalyst is more preferably cuprous chloride. By using said cuprous chloride, a more preferred reactivity may be ensured.

The catalyst may be used alone or in combination thereof, if necessary. The catalyst may be a commercially available one.

The amount of the catalyst used, per mol of the nucleophilic reagent, 3,7,11-trimethyldodecyl compound (6), is preferably 0.0001 to 1.0 mol, more preferably 0.0005 to 0.3 mol, and even more preferably 0.001 to 0.1 mol. By using said preferred amount, said more preferred amount, and said even more preferred amount, a preferred reaction rate and/or post-treatment, a more preferred reaction rate and/or post-treatment, and an even more preferred reaction rate and/or post-treatment may be ensured.

The reaction temperature of the nucleophilic addition reaction varies, depending on the nucleophilic reagent, 3,7,11-trimethyldodecyl compound (6), used, and is preferably -78 to 70°C, more preferably -20 to 50°C, and even more preferably 0 to 30°C. By using said preferred reaction temperature, said more preferred reaction temperature, and said even more preferred reaction temperature, a preferred reactivity, a more preferred reactivity, and an even more preferred reactivity may be ensured.

The reaction time of the nucleophilic addition reaction varies, depending on the solvent used and/or the production scale, and is preferably 0.5 to 100 hours. By using said preferred reaction time, a preferred reactivity may be ensured.

Thus, according to embodiments of the present invention, a 1-arenesulfonyloxy-5-haloalkane compound (3) may be easily and efficiently prepared. According to embodiments of the present invention, the sex pheromone of the Rice moth (scientific name: *Corcyra cephalonica*), 6,10,14-trimethyl-2-pentadecanol (8), may be prepared from a 1-arenesulfonyloxy-5-haloalkane compound (3) when R¹ = CH₃, i.e., a 1-arenesulfonyloxy-5-halo-3-methylpentane compound (3: R¹ = CH₃), in fewer steps with high ease of preparation.

### EXAMPLES

The present invention will be described with reference to the following Examples. It should be noted that the present invention is not limited to or by the Examples.

The term "purity" as used herein means an area percentage in gas chromatography (GC), unless otherwise specified. The term "product ratio" means a ratio of area percentages in GC. The term "yield" is calculated from the area percentages determined by GC.

In the Examples, monitoring of the reactions and calculation of the yields were carried out in the following GC conditions.

GC conditions: GC: Capillary gas chromatograph GC-2014 (Shimadzu Corporation); column: DB-5, 0.25 µm x 0.25 mmϕ x 30 m; carrier gas: He (1.55 mL/min), detector: FID; column temperature: 100°C, elevated in a rate of 5°C/min, and up to 230°C.

The yield was calculated according to the following equation in consideration of purities (% GC) of a starting material and a product. 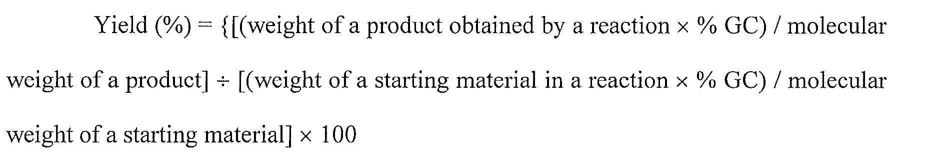 Yield (%) = {[(weight of a product obtained by a reaction × % GC) / molecular weight of a product] ÷ [(weight of a starting material in a reaction × % GC) / molecular weight of a starting material] × 100

THF represents tetrahydrofuran, MTHP represents 4-methyltetrahydropyran, Ph represents a phenyl group, and Ts represents a tosyl group.

### Example 1: Preparation of 1-benzenesulfonyloxy-5-chloro-3-methylpentane (3: R¹ = CH₃, R² = H, X¹ = Cl)

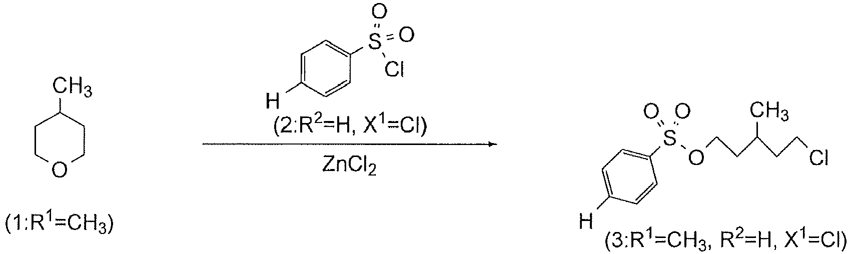

Zinc chloride (2.18 g, 0.016 mol) and benzenesulfonyl chloride (35.32 g, 0.20 mol) were placed in a reactor at a room temperature and stirred for 36 minutes at 85 to 90°C. 4-Methyltetrahydropyran (1: R¹ = CH₃) (22.04 g, 0.22 mol, purity 100%) was then added dropwise to the reactor at 85 to 95°C. After the completion of the dropwise addition, the reaction mixture was stirred for 9 hours at 85 to 90°C. Brine (prepared from sodium chloride (25.30 g) and water (80.00 g)) was then added to the reaction mixture, then 4-methyltetrahydropyran (20.00 g) was added, then methanol (20.00 g) was added, and then the reaction mixture was stirred for 30 minutes. After the completion of the stirring, the reaction mixture was transferred to a separatory funnel. The reaction mixture was left to stand, and the degree of separation of the organic layer and the aqueous layer was verified after 1 minute and then after 30 minutes. The separation was good at both 1 minute and 30 minutes, and a boundary layer was not visible (not exist). After the reaction mixture was left to stand for 30 minutes, phase separation was carried out, and then the organic layer thus obtained was concentrated at a reduced pressure to form 1-benzenesulfonyloxy-5-chloro-3-methylpentane (3: R¹ = CH₃, R² = H, X¹ = Cl) (51.32 g, 0.16 mol, purity 88.84%) with a yield of 82.36%. The concentrated solution of 1-benzenesulfonyloxy-5-chloro-3-methylpentane (3: R¹ = CH₃, R² = H, X¹ = Cl) thus obtained had no solids or residue, and the liquid was not cloudy.

The following is the spectrum data of the 1-benzenesulfonyloxy-5-chloro-3-methylpentane (3: R¹ = CH₃, R² = H, X¹ = Cl) thus obtained.

Nuclear magnetic resonance spectrum: ¹H-NMR (500 MHz, CDCl₃): δ = 0.85 (3H, d, J = 6.5 Hz), 1.40-1.86 (5H, m), 3.42-3.62 (2H, m), 4.05-4.14 (2H, m), 7.56 (2H, t-like, J = 7.3 Hz), 7.66 (1H, tt, J = 7.7 Hz, 1.6 Hz), 7.91 (2H, d-like, J = 7.8 Hz); ¹³C-NMR (125 MHz, CDCl₃): δ = 18.41, 26.92, 35.30, 39.16, 42.50, 68.62, 127.80, 129.23, 133.73, 136.07.

Mass spectrum: EI-mass spectrum (70 eV): m/z 244 (M⁺-32), 229, 213, 199, 185, 159, 141, 118, 94, 77, 55, 41.

Infrared absorption spectrum: (D-ATR): v = 2963, 2932, 1449, 1360, 1187, 1097, 945, 893, 755, 689, 590.

### Example 2: Preparation of 1-benzenesulfonyloxy-5-chloro-3-methylpentane (3: R¹ = CH₃, R² = H, X¹ = Cl)

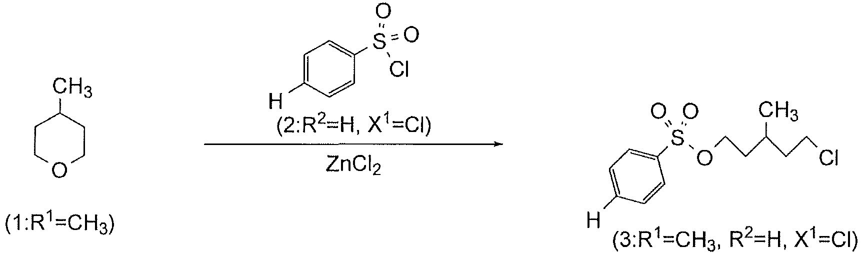

A reaction mixture was prepared identically to Example 1, including the scale, up to reaction completion at 9 hours, then brine (prepared from sodium chloride (25.30 g) and water (80.00 g)) was added to the reaction mixture, then 4-methyltetrahydropyran (20.00 g) was added, and then the reaction mixture was stirred for 30 minutes. After the completion of the stirring, the reaction mixture was transferred to a separatory funnel. Specifically, unlike Example 1, methanol (20.00 g) was not added. The reaction mixture was left to stand, and the degree of separation of the organic layer and the aqueous layer was verified after 1 minute and then after 30 minutes. The separation was unacceptable at both 1 minute and 30 minutes, and a murky boundary layer remained. After the reaction mixture was left to stand for 30 minutes, the boundary layer was left in the organic layer side, and phase separation was carried out. The organic layer thus obtained was then concentrated at a reduced pressure to form 51.74 g of 1-benzenesulfonyloxy-5-chloro-3-methylpentane (3: R¹ = CH₃, R² = H, X¹ = Cl). However, the liquid thus obtained was a suspension with a large amount of precipitated solids, which appeared to be salts of reaction products, and so filtration with a glass filter was carried out to form 1-benzenesulfonyloxy-5-chloro-3-methylpentane (3: R¹ = CH₃, R² = H, X¹ = Cl) (50.73 g, 0.16 mol, purity 90.01%) with a yield of 82.49%.

Various spectrum data of the 1-benzenesulfonyloxy-5-chloro-3-methylpentane (3: R¹ = CH₃, R² = H, X¹ = Cl) thus obtained were the same as those of Example 1.

### Example 3: Preparation of 1-chloro-3-methyl-5-(p-toluenesulfonyloxy)pentane (3: R¹ = CH₃, R² = CH₃, X¹ = Cl)

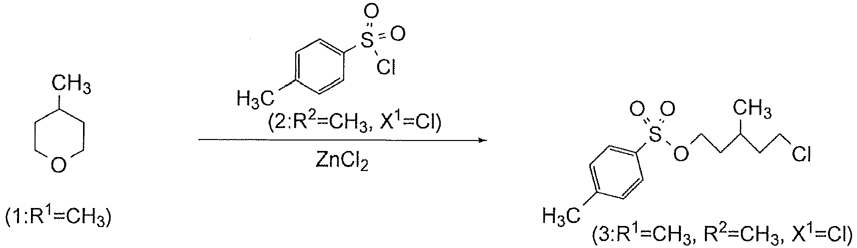

Reactions similar to those of Example 1 were carried out, with the proviso that benzenesulfonyl chloride (35.32 g, 0.20 mol) was replaced with p-toluenesulfonyl chloride (38.13 g, 0.20 mol). The reaction mixture was transferred to a separatory funnel and then left to stand. The degree of separation of the organic layer and the aqueous layer was then verified after 1 minute and then after 30 minutes. The separation was good at both 1 minute and 30 minutes, and a boundary layer was not visible. After the reaction mixture was left to stand for 30 minutes, phase separation was carried out, and then the organic layer thus obtained was concentrated at a reduced pressure to form 1-chloro-3-methyl-5-(*p*-toluenesulfonyloxy)pentane (3: R¹ = CH₃, R² = CH₃, X¹ = Cl) (53.41 g, 0.17 mol, purity 93.60%) with a yield of 85.96%. The concentrated solution of 1-chloro-3-methyl-5-(*p*-toluenesulfonyloxy)pentane (3: R¹ = CH₃, R² = CH₃, X¹ = Cl) thus obtained had no solids or residue, and the liquid was not cloudy.

The following is the spectrum data of the 1-chloro-3-methyl-5-(p-toluenesulfonyloxy)pentane (3: R¹ = CH₃, R² = CH₃, X¹ = Cl) thus obtained.

Nuclear magnetic resonance spectrum: ¹H-NMR (500 MHz, CDCl₃): δ = 0.85 (3H, d, J = 6.5 Hz), 1.40-1.87 (5H, m), 2.45 (3H, s), 3.42-3.62 (2H, m), 4.02-4.11 (2H, m), 7.35 (2H, d, J = 8.0 Hz), 7.79 (2H, d, J = 8.0 Hz); ¹³C-NMR (125 MHz, CDCl₃): δ = 18.43, 21.60, 26.93, 35.28, 39.15, 42.53, 68.38, 127.84, 129.83, 132.98, 144.78.

Mass spectrum: EI-mass spectrum (70 eV): m/z 290 (M⁺), 227, 213, 199, 173, 155, 118, 91, 69, 55, 41.

Infrared absorption spectrum: (D-ATR): v = 2963, 2931, 1598, 1457, 1359, 1176, 1097, 946, 891, 816, 664, 555.

### Example 4: Preparation of 1-chloro-3-methyl-5-(p-toluenesulfonyloxy)pentane (3: R¹ = CH₃, R² = CH₃, X¹ = Cl)

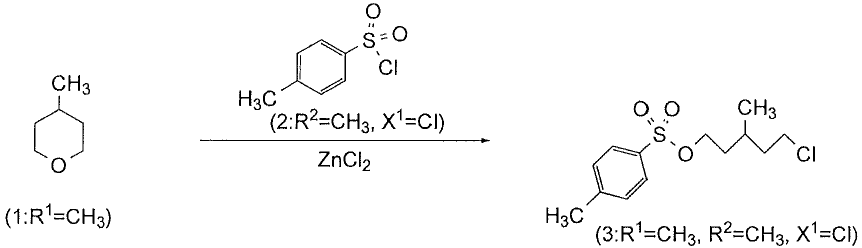

A reaction mixture was prepared identically to Example 3, including the scale, up to reaction completion at 9 hours, then brine (prepared from sodium chloride (25.30 g) and water (80.00 g)) was added to the reaction mixture, then 4-methyltetrahydropyran (20.00 g) was added, and then the reaction mixture was stirred for 30 minutes. After the completion of the stirring, the reaction mixture was transferred to a separatory funnel. Specifically, unlike Example 3, methanol (20.00 g) was not added. The reaction mixture was left to stand, and the degree of separation of the organic layer and the aqueous layer was verified after 1 minute and then after 30 minutes. The separation was unacceptable at both 1 minute and 30 minutes, and a murky boundary layer remained. After the reaction mixture was left to stand for 30 minutes, the boundary layer was left in the organic layer side, and phase separation was carried out. The organic layer thus obtained was then concentrated at a reduced pressure to form 52.30 g of 1-chloro-3-methyl-5-(p-toluenesulfonyloxy)pentane (3: R¹ = CH₃, R² = CH₃, X¹ = Cl). However, the liquid thus obtained was suspended with a large amount of precipitated solids, which appeared to be salts of reaction products, and so filtration with a glass filter was carried out to form 1-chloro-3-methyl-5-(p-toluenesulfonyloxy)pentane (3: R¹ = CH₃, R² = CH₃, X¹ = Cl) (50.17 g, 0.16 mol, purity 92.91%) with a yield of 80.15%.

Various spectrum data of the 1-chloro-3-methyl-5-(p-toluenesulfonyloxy)pentane (3: R¹ = CH₃, R² = CH₃, X¹ = Cl) thus obtained were the same as those of Example 3.

### Example 5: Preparation of 1-benzenesulfonyloxy-5-chloropentane (3: R¹ = H, R² = H, X¹ = Cl)

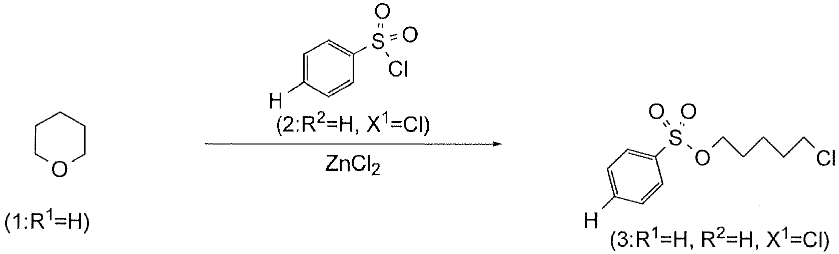

Reactions similar to those of Example 1 were carried out, with the proviso that 4-methyltetrahydropyran (1: R¹ = CH₃) (22.04 g, 0.22 mol, purity 100%) was replaced with tetrahydropyran (1: R¹ = H) (18.95 g, 0.22 mol, purity 100%), and 4-methyltetrahydropyran (20.00 g) to be added after the quenching of the reaction was replaced with tetrahydropyran (20.00 g). The reaction mixture was transferred to a separatory funnel and then left to stand. The degree of separation of the organic layer and the aqueous layer was then verified after 1 minute and then after 30 minutes. The separation was good at both 1 minute and 30 minutes, and a boundary layer was not visible. After the reaction mixture was left to stand for 30 minutes, phase separation was carried out, and then the organic layer thus obtained was concentrated at a reduced pressure to form 1-benzenesulfonyloxy-5-chloropentane (3: R¹ = H, R² = H, X¹ = Cl) (47.77 g, 0.16 mol, purity 88.80%) with a yield of 80.72%. The concentrated solution of 1-benzenesulfonyloxy-5-chloropentane (3: R¹ = H, R² = H, X¹ = Cl) thus obtained had no solids or residue, and the liquid was not cloudy.

The following is the spectrum data of the 1-benzenesulfonyloxy-5-chloropentane (3: R¹ = H, R² = H, X¹ = Cl) thus obtained.

Nuclear magnetic resonance spectrum: ¹H-NMR (500 MHz, CDCl₃): δ = 1.42-1.50 (2H, m), 1.64-1.75 (4H, m), 3.47 (2H, t, J = 6.5 Hz), 4.06 (2H, t, J = 6.5 Hz), 7.56 (2H, t-like, J = 7.3 Hz), 7.66 (1H, ddt, J = 7.3 Hz, 7.3 Hz, 1.2 Hz), 7.89-7.92 (2H, m); ¹³C-NMR(125 MHz, CDCl₃): δ = 18.43, 21.60, 26.93, 35.28, 39.15, 42.53, 68.38, 127.84, 129.83, 132.98, 144.78.

Mass spectrum: EI-mass spectrum (70 eV): m/z 262 (M⁺), 213, 159, 141, 94, 77, 51.

Infrared absorption spectrum: (D-ATR): v = 2956, 1448, 1361, 1187, 1097, 947, 822, 756, 689, 590.

### Example 6: Preparation of 1-benzenesulfonyloxy-5-chloropentane (3: R¹ = H, R² = H, X¹ = Cl)

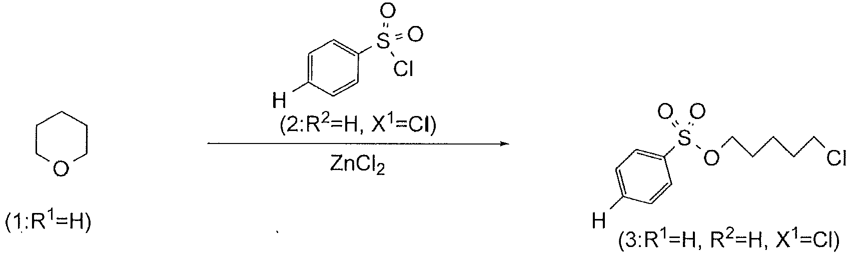

A reaction mixture was prepared identically to Example 5, including the scale, up to reaction completion at 9 hours, then brine (prepared from sodium chloride (25.30 g) and water (80.00 g)) was added to the reaction mixture, then tetrahydropyran (20.00 g) was added, and then the reaction mixture was stirred for 30 minutes. After the completion of the stirring, the reaction mixture was transferred to a separatory funnel. Specifically, unlike Example 5, methanol (20.00 g) was not added. The reaction mixture was left to stand, and the degree of separation of the organic layer and the aqueous layer was verified after 1 minute and then after 30 minutes. The separation was unacceptable at both 1 minute and 30 minutes, and a murky boundary layer remained. After the reaction mixture was left to stand for 30 minutes, the boundary layer was left in the organic layer side, and phase separation was carried out. The organic layer thus obtained was then concentrated at a reduced pressure to form 47.76 g of 1-benzenesulfonyloxy-5-chloropentane (3: R¹ = H, R² = H, X¹ = Cl). However, the liquid thus obtained was a suspension with a large amount of solids, which appeared to be precipitated salts of reaction products, and so filtration with a glass filter was carried out to form 1-benzenesulfonyloxy-5-chloropentane (3: R¹ = H, R² = H, X¹ = Cl) (46.88 g, 0.16 mol, purity 92.91%) with a yield of 82.89%.

Various spectrum data of the 1-benzenesulfonyloxy-5-chloropentane (3: R¹ = H, R² = H, X¹ = Cl) thus obtained were the same as those of Example 5.

### Example 7: Preparation of 1-chloro-5-(p-toluenesulfonyloxy)pentane (3: R¹ = H, R² = CH₃, X¹ = Cl)

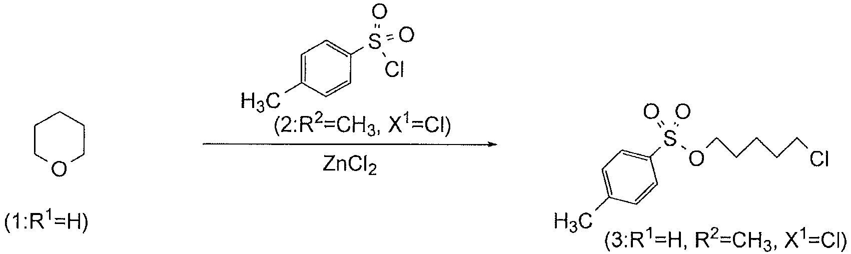

Reactions similar to those of Example 5 were carried out, with the proviso that benzenesulfonyl chloride (35.32 g, 0.20 mol) was replaced with p-toluenesulfonyl chloride (38.13 g, 0.20 mol). The reaction mixture was transferred to a separatory funnel and left to stand, and the degree of separation of the organic layer and the aqueous layer was verified after 1 minute and then after 30 minutes. The separation was good at both 1 minute and 30 minutes. A boundary layer was not visible. After the reaction mixture was left to stand for 30 minutes, phase separation was carried out, and then the organic layer thus obtained was concentrated at a reduced pressure to form 1-chloro-5-(*p-*toluenesulfonyloxy)pentane (3: R¹ = H, R² = CH₃, X¹ = Cl) (50.49 g, 0.17 mol, purity 92.16%) with a yield of 84.06%. The concentrated solution of 1-chloro-5-(*p-*toluenesulfonyloxy)pentane (3: R¹ = H, R² = CH₃, X¹ = Cl) thus obtained had no solids or residue, and the liquid was not cloudy.

The following is the spectrum data of the 1-chloro-5-(p-toluenesulfonyloxy)pentane (3: R¹ = H, R² = CH₃, X¹ = Cl) thus obtained.

Nuclear magnetic resonance spectrum: ¹H-NMR (500 MHz, CDCl₃): δ = 1.42-1.50 (2H, m), 1.67 (2H, tt, J = 6.5 Hz, 6.5 Hz), 1.71 (2H, tt, J = 6.5 Hz, 6.5 Hz), 2.45 (3H, s), 3.47 (2H, t, J = 6.5 Hz), 4.03 (2H, t, J = 6.5 Hz), 7.35 (2H, d, J = 8.4 Hz), 7.78 (2H, d, J = 8.4 Hz); ¹³C-NMR (125 MHz, CDCl₃): δ = 21.60, 22.77, 28.10, 31.75, 44.51, 70.09, 127.83, 129.82, 132.99, 144.77.

Mass spectrum: EI-mass spectrum (70 eV): m/z 276 (M⁺), 173, 155, 121, 107, 91, 68.

Infrared absorption spectrum: (D-ATR): v = 2956, 1598, 1456, 1359, 1177, 1098, 948, 816, 664, 555.

### Example 8: Preparation of 1-chloro-5-(p-toluenesulfonyloxy)pentane (3: R¹ = H, R² = CH₃, X¹ = Cl)

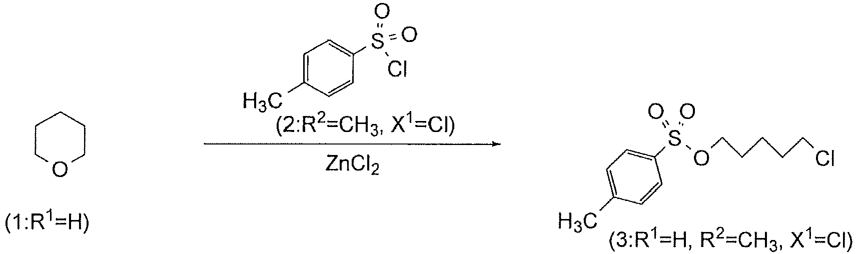

A reaction mixture was prepared identically to Example 7, including the scale, up to reaction completion at 9 hours, then brine (prepared from sodium chloride (25.30 g) and water (80.00 g)) was added to the reaction mixture, then tetrahydropyran (20.00 g) was added, and then the reaction mixture was stirred for 30 minutes. After the completion of the stirring, the reaction mixture was transferred to a separatory funnel. Specifically, unlike Example 7, methanol (20.00 g) was not added. The reaction mixture was left to stand, and the degree of separation of the organic layer and the aqueous layer was verified after 1 minute and then after 30 minutes. The separation was unacceptable at both 1 minute and 30 minutes, and a murky boundary layer remained. After the reaction mixture was left to stand for 30 minutes, the boundary layer was left in the organic layer side, and phase separation was carried out. The organic layer thus obtained was then concentrated at a reduced pressure to form 52.73 g of 1-chloro-5-(p-toluenesulfonyloxy)pentane (3: R¹ = H, R² = CH₃, X¹ = Cl). However, the liquid thus obtained was a suspension with a large amount of solids, which appeared to be precipitated salts of reaction products, and so filtration with a glass filter was carried out to form 1-chloro-5-(p-toluenesulfonyloxy)pentane (3: R¹ = H, R² = CH₃, X¹ = Cl) (50.03 g, 0.17 mol, purity 93.22%) with a yield of 84.25%.

Various spectrum data of the 1-chloro-5-(*p*-toluenesulfonyloxy)pentane (3: R¹ = H, R² = CH₃, X¹ = Cl) thus obtained were the same as those of Example 7.

### Example 9: Preparation of 1-chloro-3,7,11-trimethyldodecane (5: X¹ = Cl)

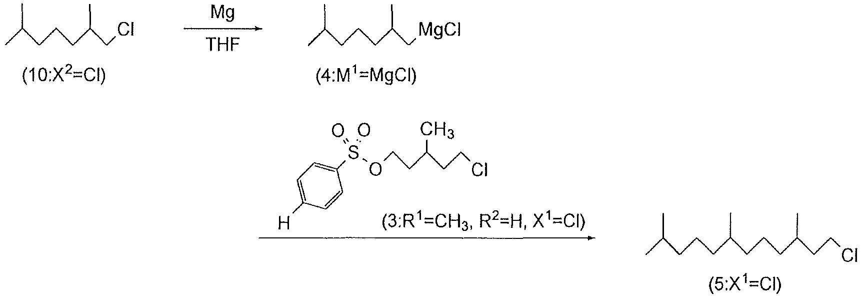

Magnesium (32.89 g, 1.35 gram atoms) and tetrahydrofuran (386.70 g) were placed in a reactor at a room temperature and stirred for 11 minutes at 60 to 65°C. 1-Chloro-2,6-dimethylheptane (10: X² = Cl) (209.75 g, 1.29 mol, purity 100%) was then added dropwise to the reactor at 60 to 75°C. After the completion of the dropwise addition, the reaction mixture was stirred for 3 hours at 75 to 80°C to form 2,6-dimethylheptylmagnesium chloride (4: M¹ = MgCl).

Next, cupric chloride (0.81 g, 0.0064 mol), lithium chloride (0.51 g, 0.013 mol), tetrahydrofuran (400.00 g), and 1-benzenesulfonyloxy-5-chloro-3-methylpentane (3: R¹ = CH₃, R² = H, X¹ = Cl) (375.23 g, 1.22 mol, purity 84.09%) prepared as in Example 1 were placed in another reactor, and then the aforesaid 2,6-dimethylheptylmagnesium chloride (4: M¹ = MgCl) was added dropwise to the other reactor at 20 to 30°C. After the completion of the dropwise addition, the reaction mixture was stirred for 3 hours at 20 to 30°C. Methanol (187.50 g) was then added to the reaction mixture, then water (86.67 g) was added, followed by phase separation. The organic layer thus obtained was concentrated at a reduced pressure, and the resulting concentrate was distilled at a reduced pressure to form 1-chloro-3,7,11-trimethyldodecane (5: X¹ = Cl) (249.64 g, 0.96 mol, purity 96.17%, b.p. = 128.4 to 131.1°C/0.12 kPa (0.92 mmHg)) with a yield of 78.78%.

The following is the spectrum data of the 1-chloro-3,7,11-trimethyldodecane (5: X¹ = Cl) thus obtained.

Nuclear magnetic resonance spectrum: ¹H-NMR (500 MHz, CDCl₃): δ = 0.83-0.91 (12H, m), 1.02-1.42 (13H, m), 1.48-1.69 (3H, m), 1.75-1.84 (1H, m), 3.50-3.62 (2H, m); ¹³C-NMR (125 MHz, CDCl₃): δ = 19.05, 19.12, 19.66, 19.72, 22.61, 22.71, 24.23, 24.79, 24.81, 27.97, 30.38, 30.41, 32.74, 32.76, 36.90, 36.96, 37.23, 37.25, 37.28, 37.36, 39.35, 39.77, 39.85, 43.37.

Mass spectrum: EI-mass spectrum (70 eV): m/z 246 (M⁺), 231, 203, 175, 161, 147, 133, 113, 97, 83, 71, 57, 43.

Infrared absorption spectrum: (D-ATR): v = 2955, 2926, 1463, 1379, 730, 660.

### Example 10: Preparation of 6,10,14-trimethyl-2-pentadecanol (8)

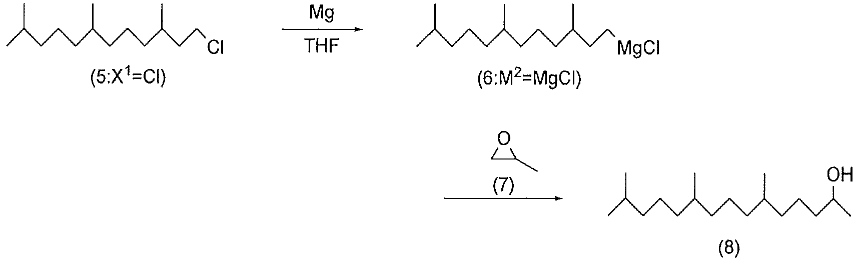

Magnesium (30.34 g, 1.25 gram atoms) and tetrahydrofuran (356.70 g) were placed in a reactor at a room temperature and then stirred for 22 minutes at 60 to 65°C. 1-Chloro-3,7,11-trimethyldodecane (5: X¹ = Cl) (307.68 g, 1.19 mol, purity 96.17%) prepared as in Example 9 was then added dropwise to the reactor at 60 to 75°C. After the completion of the dropwise addition, the reaction mixture was stirred for 2.5 hours at 75 to 80°C to form 3,7,11-trimethyldodecylmagnesium chloride (6: M² = MgCl).

The internal temperature of the aforesaid reactor was then cooled to 0 to 10°C, then cuprous chloride (0.25 g, 0.0024 mol, purity 95%) was added to the reactor, and then propylene oxide (79.42 g, 1.36 mol) was added dropwise to the reactor at 5 to 30°C. After the completion of the dropwise addition, the reaction mixture was stirred for 2 hours at 15 to 25°C. An aqueous solution of acetic acid (prepared from acetic acid (162.26 g) and water (327.59 g)) was then added to the reaction mixture, followed by phase separation. The organic layer thus obtained was then concentrated at a reduced pressure, and the resulting concentrate was distilled at a reduced pressure to form 6,10,14-trimethyl-2-pentadecanol (8) (288.54 g, 1.06 mol, purity 99.56%, b.p. = 145.0 to 153.1°C/0.12 kPa (0.90 mmHg)) with a yield of 89.33%.

The following is the spectrum data of the 6,10,14-trimethyl-2-pentadecanol (8) thus obtained.

Nuclear magnetic resonance spectrum: ¹H-NMR (500 MHz, CDCl₃): δ = 0.85 (12H, dt, J = 6.1 Hz, 6.1 Hz), 1.00-1.16 (6H, m), 1.18 (3H, d, J = 6.1 Hz), 1.20-1.47 (15H, m), 1.52 (1H, dq, J = 6.5 Hz, 6.5 Hz), 3.79 (1H, sext-like, J = 6.1 Hz); ¹³C-NMR (125 MHz, CDCl₃): δ = 19.60, 19.67, 19.73, 22.61, 22.70, 23.21, 23.23, 23.25, 23.47, 23.51, 24.45, 24.78, 24.80, 27.96, 32.72, 32.75, 32.78, 36.99, 37.02, 37.08, 37.11, 37.27, 37.30, 37.32, 37.37, 37.42, 39.35, 39.69, 39.71, 68.17, 68.21.

Mass spectrum: EI-mass spectrum (70 eV): m/z 269 (M⁺-1), 252, 224, 182, 167, 140, 126, 111, 97, 71, 57, 43.

Infrared absorption spectrum: (D-ATR): v = 3330, 2954, 2926, 1463, 1377, 1121, 938.

## Claims

1. A process for preparing a 1-arenesulfonyloxy-5-haloalkane compound of the following general formula (3): 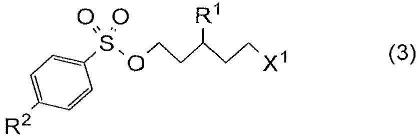
wherein R¹ and R² represent, independently of each other, a hydrogen atom or a methyl group, and X¹ represents a halogen atom,
the process comprising a step of:
subjecting a tetrahydropyran compound of the following general formula (1): 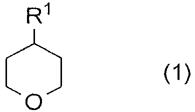
wherein R¹ represents a hydrogen atom or a methyl group,
to a ring-opening reaction with an arenesulfonyl halide compound of the following general formula (2): 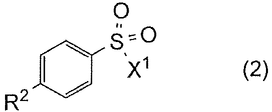
wherein R² represents a hydrogen atom or a methyl group, and X¹ represents a halogen atom,
in the presence of zinc chloride to form the aforesaid 1-arenesulfonyloxy-5-haloalkane compound (3).

2. A process for preparing a 1-halo-3,7,11-trimethyldodecane compound of the following general formula (5): 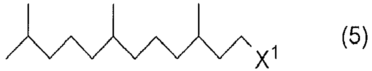
wherein X¹ represents a halogen atom,
the process comprising steps of:
the process according to claim 1 for preparing a 1-arenesulfonyloxy-5-haloalkane compound (3), wherein R¹ = CH₃ (hereinafter, referred to as a 1-arenesulfonyloxy-5-haloalkane compound (3: R¹ = CH₃)) from a tetrahydropyran compound (1) wherein R¹ = CH₃, and
subjecting a nucleophilic reagent, 2,6-dimethylheptyl compound, of the following general formula (4): 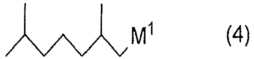
wherein M¹ represents Li, MgZ¹, CuZ¹, or CuLiZ¹, and Z¹ represents a halogen atom or a 2,6-dimethylheptyl group,
to a coupling reaction with the 1-arenesulfonyloxy-5-halo-3-methylpentane compound (3: R¹ = CH₃)
to form the aforesaid 1-halo-3,7,11-trimethyldodecane compound (5).

3. A process for preparing 6,10,14-trimethyl-2-pentadecanol of the following formula (8): 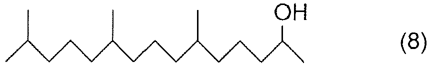
the process comprising steps of:
the process according to claim 1 for preparing the aforesaid 1-arenesulfonyloxy-5-haloalkane compound (3), wherein R¹ = CH₃ (hereinafter, referred to as the 1-arenesulfonyloxy-5-haloalkane compound (3: R¹ = CH₃)) from a tetrahydropyran compound (1) wherein R¹ = CH₃,
subjecting a nucleophilic reagent, 2,6-dimethylheptyl compound, of the following general formula (4): 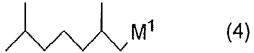
wherein M¹ represents Li, MgZ¹, CuZ¹, or CuLiZ¹, and Z¹ represents a halogen atom or a 2,6-dimethylheptyl group,
to a coupling reaction with the 1-arenesulfonyloxy-5-halo-3-methylpentane compound (3: R¹ = CH₃) to form a 1-halo-3,7,11-trimethyldodecane compound of the following general formula (5): 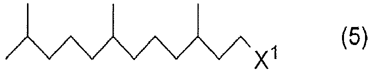
wherein X¹ represents a halogen atom,
converting the 1-halo-3,7,11-trimethyldodecane compound (5) thus obtained into a nucleophilic reagent, 3,7,11-trimethyldodecyl compound, of the following general formula (6): 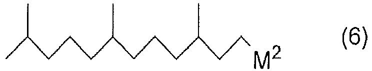
wherein M² represents Li, MgZ², CuZ², or CuLiZ², and Z² represents a halogen atom or a 3,7,11-trimethyldodecyl group,
and
subjecting the nucleophilic reagent, 3,7,11-trimethyldodecyl compound (6) thus obtained, to a nucleophilic addition reaction with propylene oxide of the following formula (7): 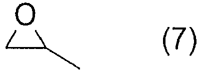
to form the aforesaid 6,10,14-trimethyl-2-pentadecanol (8).
